# EUROPEAN PATENT SPECIFICATION

(11) **EP 1 656 969 B1**
(45) Date of publication and mention of the grant of the patent: **30.03.2011**
(21) Application number: 04026521.7
(22) Date of filing: 09.11.2004
(51) Int. Cl.: A61Q 5/06, A61Q 5/00, A61Q 5/12, A61K 8/365, A61K 8/362

(54) **Conditioning and coloring composition for Hair**
Konditionierende und Färbemittel fur die Haare
Composition conditionnante et colorante pour les cheveux

(43) Date of publication of application: 17.05.2006
(73) Proprietor: KPSS-Kao Professional Salon Services GmbH, 64297 Darmstadt (DE)
(72) Inventor: Molenda, Michael, 60598 Frankfurt (DE); Golinski, Frank, Dr., 64372 Ober-Ramstadt (DE); Herzog-Renker, Beate, 64291 Darmstadt (DE)

(56) References cited:
- EP-A- 1 287 811
- EP-A- 1 366 754
- EP-A- 1 428 497
- WO-A-95/15144
- WO-A-03/086335
- WO-A-2004/054526

## Description

The present invention concerns a conditioning and colouring composition for hair with optimum conditioning effects especially shines, softness and manageability.

Coloring and conditioning compositions have been known for years and have been proven to be very successful on the market.

Such compositions customarily comprise hair coloring direct dyestuffs together with the at least one hair conditioning compound, especially of cationic types.

Although these products are proven as such to be satisfying consumer needs, it is still desirable to improve their efficiency especially in terms of hair shine, softness and manageability together with good colour enhancing ability, and certainly with other hair conditioning properties in particular with regard to volume and body, and combability.

Thus, the object of the present invention is providing a conditioning composition showing excellent colour enhancing and conditioning abilities together with excellent shine enhancing effect, improving excellently manageability and softness of hair, at the same time having optimum conditioning effects on hair, which satisfies consumer expectations in terms of combability, volume and body, and nice feeling on touching hair.

It has surprisingly been found out that a hair conditioning composition comprising at least one hair conditioning compound, at least one cationic hair dye and at least one hydroxycarboxylic acid and/or dicarboxylic acid, show optimum performance in colour enhancing and especially surprisingly excellently superior performance in hair shine improving, excellently improving hair manageability and softness of hair. The compositions of the present invention also improve combability, volume and body of hair. After using compositions of the present invention, hair feels nicer when touching. The effects mentioned are more pronounced on repeated usage and especially together with a shampoo composition suitable therefore.

EP 1174112 discloses hair cosmetic compositions comprising organic acid, organic solvent, cationic surfactant and higher alcohol and having pH in the range of 2 to 6 for improving hair shine. Additionally, WO 2004/047777 discloses leave-in compositions for hair comprising malic and lactic acids and organic solvents for improving shine, setting and touch feeling. Both documents are silent on color enhancing and conditioning compositions comprising cationic direct dyes.

The pH of the compositions according to the present invention is suitably below 4.5 and preferably in the range of 2.0 to 4.0, more preferably 2.5 to 4.0, most preferably 2.5 to 3.80.

The pH of the compositions is adjusted with hydroxycarboxilic acids and/or dicarboxylic acids. In those cases where selected hydroxycarboxylic acid and/or dicarboxylic acid concentration is not enough to reach the selected pH, other organic and inorganic acids may as well be used to adjust pH to the required value. The hydroxycarboxilic acids useful in the compositions of the present invention are lactic acid, glycolic acid, hydroxyacrylic acid, glyceric acid, malic acid and tartaric acid and of the dicarboxylic acids are malonic acid, succinic acid, glutaric acid, adipic acid, maleic acid, fumaric acid and phtalic acid.

Compositions according to present invention in principal comprise at least one hydroxycarboxylic acid and/or dicarboxylic acid. Combinations of two or more hydroxycarboxylic acids and/or dicarboxylic acids are also within the scope of the invention. It should be noted that mixture of two or more hydroxycarboxylic acid and dicarboxylic acid comprising compositions are also within the scope of the present invention. Especially preferred hydroxycarboxylic acids are the lactic and malic acids. Malic acid is also a dicarboxylic acid. The most preferred hydroxycarboxylic acid and/or dicarboxylic acid is the malic acid.

Total hydroxycarboxylic acid and/or dicarboxylic acid concentration in the composition of the present invention varies in the range form 0.1 to 5% by weight, preferably 0.25 to 3% by weight, more preferably 0.5 to 3% by weight and most preferably 0.75 to 3% by weight. In a preferred embodiment of the invention, the compositions of the present invention comprise at least 0.5% malic acid.

According to the invention, conditioning and colouring composition comprises at least one direct acting cationic dyestuff. Suitable cationic dyestuffs are in principal those available on the market for hair colouring applications. Some examples to those are:
Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14 and Basic Yellow 57.
For this purpose, special reference is made to the PCT application WO 95/15144 of Ciba-Geigy AG. The cationic dyestuffs with the following chemical structures are especially preferred ones according to the present invention. 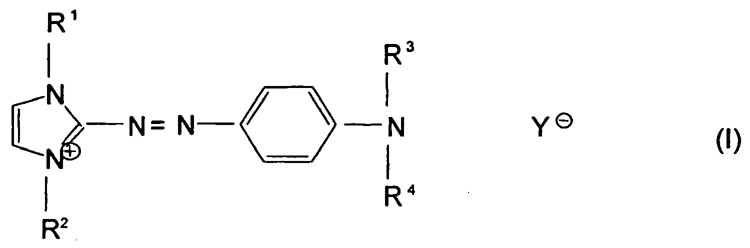 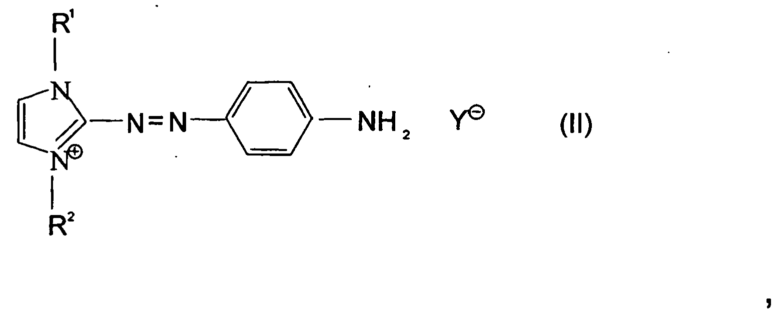 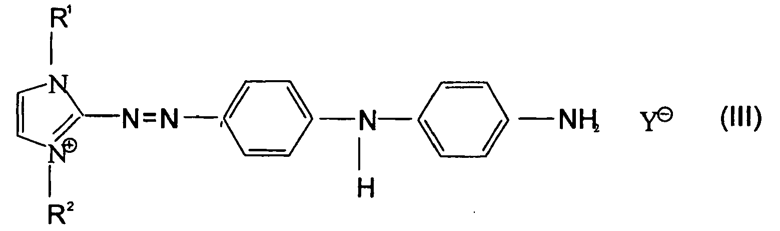 and 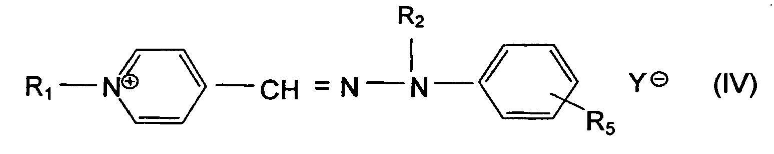 wherein R¹, R², R³ and R⁴ stand for hydrogen, a CH₃- or C₂H₅- group, R⁵ stands for hydrogen, -OCH₃ or -OC₂H₅ and Y is an anion such as chloride, bromide, methosulfate.

The most preferred cationic dyestuffs are the ones according to the formula I, where R₁, R₂, R₃ and R₄ are methyl and Y is chloride, according to formula II where R₁ and R₂ are methyl and Y is chloride and according to the formula IV, where R₁ and R₂, are methyl, R₅ is hydrogen and Y is methosulfate.

Cationic dyestuffs are included into the compositions of the present invention at a concentration of 0.0001 to 2.5%, preferably 0.0001 to 1.5% and more preferably 0.0001 to 1% by weight, calculated to total aqueous composition.

Anionic dyes may as well be used in combination with cationic direct dyes at minor quantities. The suitable ones are:
Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

According to the invention, anionic dyes may be included in minor quantities at a concentration around 25%, preferably not more than 10% of the total cationic dye content of the composition.

Additionally, the coloring compositions of the present invention may comprise neutral dyes (HC dyes), so called nitro dyes in addition to the cationic direct dyes. Concentration of those can typically be in the range of 0.0001 to 1%, preferably 0.0001 to 0.75% and more preferably 0.0001 to 0.5% by weight calculated to total aqueous composition.

Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used in combination with cationic direct-acting dyestuffs, for example henna (red or black), alkanna root, laccaic acid, indigo, logwood powder, madder root and rhubarb powder, etc.

The anionic, neutral and plant dyes are always used in combination with cationic direct dyes within the scope of the invention.

Colouring and conditioning compositions of the present invention comprise at least one hair-conditioning compound. Suitable ones are those of cationic compounds, especially cationic polymers and cationic surfactants. Oily substances and non-ionic substances can as well be suitably used as conditioners in the compositions of the present invention.

Suitable cationic polymers are those of best known with their CTFA category name Polyquatemium. Typical examples of those Polyquaternium 6, Polyquaternium 7, Polyquaternium 10, Polyquaternium 11, Polyquatemium 16, Polyquaternium 22 and Polyquaternium 28.

As well those polymers known with their CTFA category name Quatemium are suitable. Those are for example Quaternium-8, Quaternium-14, Quaternium-15, Quaternium-18, Quatemium-22, Quaternium-24, Quatemium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The most preferred cationic polymers are those of cationic cellulose derivatives, cationic guar gum derivatives, polyquaternium 6 and polyquaternium 7.

The cationic polymers also include the quaternized products of graft polymers from organopolysiloxanes and polyethyl oxazolines described in EP-A 524 612 and EP-A 640 643.

Typical concentration range for cationic polymers is 0.01 - 5% by weight, preferably 0.01 - 3.5% by weight, more preferably 0.05 - 2.5% and most preferably 0.05 - 1.5% by weight calculated to the total composition.

In one of the preferred from of the present invention, conditioning compositions comprise at least one cationic surfactant as conditioning agent and as well as emulsifier. Suitable cationic surfactants are presented with the general formula 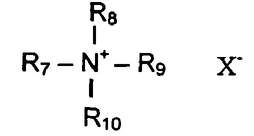 where R₇ is a saturated or unsaturated, branched or non-branched alkyl chain with 8-22 C atoms or

R₁₁ CO NH (CH₂)ₙ

where R₁₁ is saturated or unsaturated, branched or non-branched alkyl chain with 7-21 C atoms and n has value of 1 - 4,
or

R₁₂ CO O (CH₂)ₙ

where R₁₂ is saturated or unsaturated, branched or non-branched alkyl chain with 7-21 C atoms and n has value of 1 - 4, and
R₈ is H or unsaturated or saturated, branched or non-branched alkyl chain with 1 - 4 C atoms or

R₁₁ CO NH (CH₂)ₙ

or

R₁₂ CO O (CH₂)ₙ

where R₁₁, R₁₂ and n are same as above.

R₉ and R₁₀ are H or lower alkyl chain with 1 to 4 carbon atoms, and X is anion such as chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

From the above quaternary ammonium compounds disclosed with the general formula, especially to mention are those compounds known **per se** and are on the market, for example, under the trade names "Schercoquat® ", "Dehyquart® F30" and "Tetranyl® ". Use of these compounds, the so-called "esterquats", in hair care compositions is described, for example, in WO-A 93/107 48, WO-A 92/068 99 and WO-A 94/166 77, wherein, however, there is no reference made to the combinations according to the present invention and the advantageous properties thereof.

Again from the above quaternary ammonium compounds disclosed with the general formula, especially to metion are these compounds are known per se and on the market, for example, under the trade name "INCROQUAT® HO" or "OCS". Those compounds are known with a general ingredient category under "amidoquat" in the cosmetic industry.

Amido amines may as well be used as a conditioning cationic surfactant in the compositions of the present invention. Typical non-limiting example is stearamidopropylamine known with a trade name Tego Amid S18 from Goldschmidt.

Typical concentration range for cationic surfactants is 0.01 - 5% by weight, preferably 0.01 - 3.5% by weight, more preferably 0.05 - 2.5% and most preferably 0.05 - 1.5% by weight calculated to the total composition. Accordingly, the total cationic conditioning compound concentration in the compositions of the present invention varies from 0.01 to 5% by weight, preferably 0.01 to 3.5% by weight, more preferably 0.05 to 2.5% and most preferably 0.05 to 1.5% by weight calculated to the total composition

It is as well the preferred embodiment of the invention that the cationic surfactants and cationic polymers used in combination at any ratio.

Oily substances are selected from such as silicone oils, either volatile or non-volatile, natural and synthetic oils. Among silicone oils those can be added to the compositions include dimethicone, amodimethicone (a cationic silicone), dimethiconol, polydimethylsiloxane, DC fluid ranges from Dow Corning, natural oils such as olive oil, almond oil, avocado oil, weizenkeim oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

Non-ionic conditioning agents may be polyols such as glycerin, glycol and derivatives, polyethyleneglycoles known with trade names Carbowax PEG from Union Carbide and Polyox WSR range from Amerchol, polyglycerin, polyethyleneglycol mono or di fatty acid esters having general formula

R₁₃ CO (O CH₂ CH₂)ₙ OH

R₁₃ CO (O CH₂ CH₂)ₙ O OC R₁₄

where R₁₃ and R₁₄ are independent from each other saturated, unsaturated or branched or non-branched alkyl chain with 7 to 21 C atoms and n is typically 2 - 100.

The compositions according to the invention may also comprise further conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol® ", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

Amphoteric polymers are found to be useful in conditioning and colouring composition of the present invention. They are incorporated alone or in admixture with at least one additional cationic, nonionic or anionic polymer, particularly copolymers of N-octyl acrylamide, (meth)acrylic acid and tert.-butyl aminoethyl methacrylate of the type "Amphomer® "; copolymers from methacryl oylethyl betaine and alkyl methacrylates of the type "Yukaformer® ", e.g., the butyl methacrylate copolymer "Yukaformer® Am75"; copolymers from monomers containing carboxyl groups and sulfonic groups, e.g., (meth)acrylic acid and itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Colouring and conditioning compositions of the present invention can be in the form of emulsions, solutions, gels and dispersions. In the case that solutions and/or gels forms are prefered the appearance can be either with a transparent or opaque. Transparency of the composition is judged by naked eye in a transparent bottle with a thickness not more than 5 cm. As a product form, foam is as well suited when packed into a pressurized can or delivered through a pump-foamer (non-aerosol). In the case that an aerosol foam preparation is preferred, propellant gas must be added to the formulation. The suitable propellant gasses are carbondioxide, dimethylether and alkanes such as butane, propane, isobutane or their mixtures.

The emulsion type of colouring conditioners comprise additionally at least one fatty alcohol of the following formula

R₁₅-OH

where R₁₅ is a saturated or unsaturated, branched or non-branched fatty acyl chain with 8 - 24 C atoms. Concentration of fatty alcohols is usually less than 20%, preferably less than 15% by weight calculated to total composition. Typical examples to the most useful fatty alcohols are myristyl alcohol, palmityl alcohol, cetyl alcohol, stearyl alcohol, behenyl alcohol and their mixtures. As a mixed fatty alcohol the mostly used one is the cetearyl alcohol as well preferred in the compositions of the present invention.

The composition of the present invention can comprise surface-active substances as emulsifiers other than cationic surfactants disclosed above especially when an emulsion is preferred. These can be anionic and/or nonionic and/or amphoteric or zwitterionic and/or their mixtures incorporated at a concentration ranging between 0.1 - 10 %, preferably 0.1 - 7.5% and more preferably 0.1 - 5% by weight calculated to the total composition. Preferred emulsifiers are of non-ionic surfactants in addition to the cationic ones. The anionic ones are as a rule not preferred and if their presence is desirable because of any reason, those should form the very minor part, as electrostatic interactions with the cationic material especially dyes and cationic conditioners either surfactants or polymers can disturb the stability of those conditioners as well prevent colouring effect. Zwitterionic ones are also the ones preferred to lesser extent.

Suitable preferred nonionic surfactants useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Further suitable nonionic surfactants according to the invention are alkyl polyglucosides of the general formula

R₁₆-O-(R₁₇O)ₙ-Zₓ,

wherein R₁₇ is an alkyl group with 8 to 18 carbon atoms, R₁₇ is an ethylene or propylene group, Z is a saccharide group with 5 to 6 carbon atoms, n is a number from 0 to 10 and x is a number between 1 and 5.

Further nonionic surfactants are, for example, long-chain fatty acid mono- and dialkanolamides, such as coco fatty acid monoethanolamide and myristic fatty acid monoethanolamide.

Further useful nonionic surfactants are, for example, the various sorbitan esters, such as polyethylene glycol sorbitan stearic acid ester, fatty acid polyglycol esters or polycondensates of ethyleneoxide and propyleneoxide, as they are on the market, for example, under the trade name "Pluronics^{R}", as well as fatty alcohol ethoxylates.

Further suitable nonionic surfactants are amineoxides. Such amineoxides are state of the art, for example C₁₂-C₁₈- alkyl dimethyl amineoxides such as lauryl dimethyl amineoxide, C₁₂-C₁₈- alkyl amidopropyl or -ethyl amineoxides, C₁₂-C₁₈ -alkyl di(hydroxyethyl) or (hydroxypropyl) amineoxides, or also amineoxides with ethyleneoxide and/or propyleneoxide groups in the alkyl chain. Such amineoxides are on the market, for example, under the trade names "Ammonyx® ", "Aromox® " or "Genaminox® ".

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

In detail, it is possible to use betaines of the structure 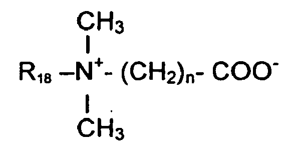 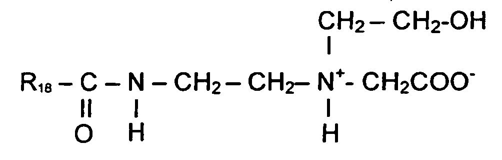 wherein R₁₈ is a C₈-C₁₈-alkyl group and n is 1 to 3; sulfobetaines of the structure 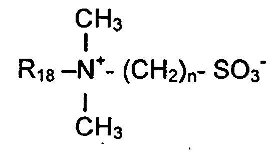 wherein R₁₈ and n are same as above;
and amidoalkyl betaines of the structure 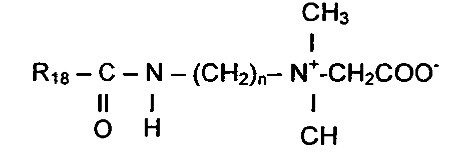 wherein R₁₈ and n are same as above.

Anionic surfactants are as a rule preferred to a lesser extend. Some of them are to mention those of sulfate, sulfonate, carboxylate and alkyl phosphate types. Examples are the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids. Such products have been known for some time and are on the market, for example, under the trade name "AKYPO® " and "AKYPO-SOFT® ".

The others to mention are C₈-C₂₀-acyl isethionates, as well as sulfofatty acids and the esters thereof.

Further anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

In another preferred form of the invention, It has been found out that in the presence of organic solvents, the effects especially the shine enhancing effect of the compositions is very much enhanced. Without being bound by any theory, it is thought that the accelerated/more pronounced effect is observed due to penetration enhancing effect of the organic solvents. Accordingly, compositions can comprise organic solvents such as ethanol, propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, propyleneglycol, poypropyleneglycols, ethyleneglycol monoethylether, ethylene glycol monobutyl ether, ethylene glycol monophenyl ether, 1-phenylethylalcohol, 2-phenylethylalcohol, o-methoxyphenol. The most preferred ones are benzyloxyethanol and polypropylene glycols. Concentration of organic solvents in the compositions should not exceed 10% by weight, preferably in the range of 0.1 to 7.5%, more preferably 0.2 to 5% by weight calculated to total composition.

The viscosity of the compositions depends on the type of application according to the invention. Emulsion type of compositions have the viscosity in the range of between about 1000 and about 50,000 mPa.s at 20°C, measured according to Brookfield or Höppler at a shear rate of 10 sec⁻¹. Whereas compositions dispensed form an aerosol and/or pump foamer should preferably be very liquid, i.e. viscosity values not more than approximately 500 mPa.s measured as given above are not appropriate.

Compositions of the present invention are used especially after shampooing and can be applied to hair either as a leave-in or as a rinse off compositions.

Furthermore, compositions of the present invention can comprise all substances customarily found in hair conditioning preparations.

Examples of such substances are natural plant extracts, complexing agents, preservatives, fragrances, moisturizers, UV filters, etc.

Natural plant extracts are incorporated usually in an amount of about 0.01 % to about 10 %, preferably 0.05 % to 7.5 %, in particular 0.1 % to 5 % by weight, calculated as dry residue thereof to the total composition. Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, cocoanut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon® " products, "Herbasol® ", "Sedaplant^{R}" and "Hexaplant^{R}". Extracts and the preparation thereof are also described in "Hagers Handbuch der pharmazeutischen Praxis", 4^{th} Ed..

Compositions of the present invention can comprise UV filters for protection of hair from environmental influences such as loss of elasticity, loss of hair colour (bleaching effect of sun light). The UV-absorbing substance is preferably selected from the following compounds: 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2.4-dihydroxybenzophenone, 2.2'.4.4'-tetrahydroxybenzophenone, 2-hydroxy-4-methoxybenzophenone and its 5-sulfonic acid or the sodium salt thereof, 2.2'-dihydroxy-4.4'-dimethoxybenzophenone, 2-hydroxy-5-chlorobenzophenone, 2.2'-dihydroxy-4-methoxybenzophenone, 2.2'-dihydroxy-4.4'-dimethoxy-5.5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher.

The preferred amount of the UV-absorber ranges from about 0.01 % to 2.5%, more preferably from 0.05 % to 1 % by weight, calculated to the total composition.

The compositions of the present invention can comprise hair-restructuring agents. The hair restructuring agents preferred are especially the ones disclosed in the German patent DE 197 51 550 C2.

One of the known hair restructuring agents is ceramide type of compound with the general formula 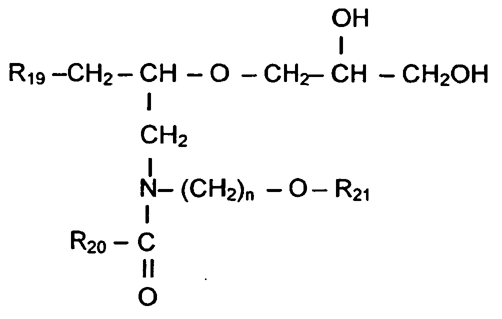 where R¹⁹ and R²⁰ are independent from each other alkyl- or. alkenyl group mit 10 to 22 carbon atoms, R²¹ is methyl, ethyl, n-propyl or isopropyl group and n is a number between 1 to 6, preferably 2 or 3.

Other preferred hair restructuring agents are fatty acids with 10 to 24 carbon atoms and especially with 16 to 24 carbon atoms.

Sterols,especially the phytosterols, are as well preferred hair restructuring agents as disclosed in the above mentioned german patent. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

The concentration of ceramide in the compositions of the present invention can be in the range of 0.01 to 2% and especially 0.01 to 1% by weight calculated to the total weight of the composition. The fatty acids may be contained at a level of 0.01 to 2.5% and epecially 0.01 to 1% by weight calculated to the total weight of the composition. Phytosterol concentration of the conditioners is less than 1% and preferably less than 0.5% by weight calculated to the total weight of the composition. It should be noted without limiting the use of those ingredients the effect of those hair restructuring ingredients is especially elevated when used in combination with penetration enhancers.

The moisturizing agents are selected from panthenol, polyols, such as glycerol, polyethylene glycols with molecular weight 200 to 20,000. The moisturizing ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

In an another preferred embodiment of the present invention is that the compositions disclosed ghere are used in combination with a cleansing composition having similar pH values and preferably comprising hydroxycarboxylic acid and/or dicarboxylic acid. In other words, the hair is cleansed firs with a composition comprising at least one cleansing surfactant and at least one hydroxycarboxylic acid and/or dicarboxylic acid subsequently treated with compositions of the present invention. This has resulted in that the all the effects especially shine and manageability is observed at elevated levels and as well as shorter usage period.

The following examples illustrate the invention.

### Example 1

### Hair treatment composition rinse-off

| | |
|---|---|
| Cetylstearylalcohol | 5.0 (% by weight) |
| Stearyltrimethylammoniumchlorid | 2.0 |
| Benzyloxyethanol | 2.5 |
| Basic red 76 | 0.1 |
| Fragrance, preservative | q.s. |
| Malic acid | 0.75 |
| Wasser | ad 100.0 |

The pH of the composition is 3.0. The color direction on hair is red.

The above composition according to present invention is tested in a comparative monadic (meaning that the users used only one composition and the comparison is made to the groups using the other comparative compositions in the same way) test with 20 volunteers aging 20 to 45 years against an identical composition (composition 1A) with a pH adjusted to 3.0 with citric acid (not within the scope of the invention). In addition, the same composition having the same pH was produced with using equal amount of lactic acid instead of malic acid (composition 1B), which is as well according to present invention. The compositions 1A and 1B were as well tested with 20 female volunteers aging 20 to 45 years. The volunteers were asked to evaluate the used shampoo composition using a questionnaire by giving marks from 1 (not good at all) to 5 (excellent). From the individual results the arithmetical average values were calculated. The evaluation was done immediately after the first usage and after 5 and 10 applications. The test period was 2 weeks being the shortest and 4 weeks being the longest. The results are presented in the table I below. It should be noted that the volunteers were asked to use for washing their hair their daily shampoo before application of the conditioner. All the shampoos used have had pH value above 5.0 as measured with a lab pH meter at ambient temperature.

**Table l: results of the home use test.**

| | **After 1 application** | | | **After 5 applications** | | | **After 10 applications** | | |
|---|---|---|---|---|---|---|---|---|---|
| | **Composition** | | | **Composition** | | | **Composition** | | |
| | **1*** | **1A**** | **1B*** | **1*** | **1A**** | **1B*** | **1*** | **1A**** | **1B*** |
| **Wet hair** | | | | | | | | | |
| Combing | 3.9 | 3.7 | 3.8 | 3.8 | 3.8 | 3.8 | 4.0 | 3.7 | 3.8 |
| Softness | 4.1 | 3.6 | 3.8 | 4.3 | 3.7 | 3.9 | 4.4 | 3.6 | 3.9 |
| Nice feel | 4.0 | 3.8 | 4.1 | 4.4 | 3.9 | 4.0 | 4.4 | 3.8 | 4.1 |
| | | | | | | | | | |

| **Dry hair** | | | | | | | | | |
|---|---|---|---|---|---|---|---|---|---|
| Combing | 3.9 | 3.6 | 3.8 | 4.1 | 3.5 | 3.8 | 4.0 | 3.6 | 3.8 |
| Softness | 3.9 | 3.6 | 3.7 | 4.2 | 3.6 | 4.0 | 4.4 | 3.7 | 4.1 |
| Nice feel | 4.1 | 3.5 | 3.8 | 4.4 | 3.8 | 4.1 | 4.3 | 3.8 | 4.0 |
| Volume | 4.3 | 3.6 | 4.1 | 4.2 | 3.7 | 4.1 | 4.3 | 3.6 | 4.1 |
| Shine | 4.4 | 3.2 | 4.1 | 4.5 | 3.2 | 4.2 | 4.6 | 3.2 | 4.4 |
| Body | 4.3 | 3.6 | 4.1 | 4.3 | 3.6 | 4.1 | 4.4 | 3.5 | 4.3 |
| Managability | 4.3 | 3.7 | 4.3 | 4.6 | 3.4 | 4.4 | 4.6 | 3.7 | 4.4 |
| Want to buy | 4.2 | 3.5 | 4.1 | 4.4 | 3.4 | 4.2 | 4.6 | 3.6 | 4.3 |

| | | | | | | | | | |
|---|---|---|---|---|---|---|---|---|---|
| *: Compositions 1 and 1B are according to the invention **: Composition 1A is not part of the invention - comparative composition. | | | | | | | | | |

From the results in the table above, it is clear that the inventive compositions 1 and 1B showed superior performance over the comparative composition 1A in all items listed. The superior performance of the inventive compositions after repeated applications (5 and 10) was more pronounced. In addition, the composition 1 comprising malic acid and having pH 3.5 was superior to both lactic acid comprising composition and to the comparative composition. Lactic acid comprising composition 1B (pH 3.5 - inventive) showed better performance than comparative composition.

The same test above was carried out with an additional 20 female aging 20 to 45 and asked to use before using the above conditioner composition - Composition 1 - a shampoo composition with the following formulation.

### Shampoo composition

| | |
|---|---|
| Sodium lauryl ether carboxylate (10EO) | 5.0 (% by wt.) |
| C₈-C₂₂-Alkyl glucoside (P.D.:∼ 1.5) | 5.0 |
| Cocoamidopropyl betaine | 5.0 |
| Cetyl PEG/PPG-10/1 dimethicone | 0.5 |
| Cationic polymer (Polyquaternium-7) | 0.1 |
| Sodium benzoate | 0.6 |
| Benzyloxyethanol | 0.5 |
| Peach oil | 0.1 |
| Perfume | 0.7 |
| PEG-60-hydrogenated castor oil | 0.5 |
| PEG-18 Glyceryl cocoate/oleate | 1.0 |
| Basic red 76 | 0.09 |
| Basic red 51 | 0.06 |
| Malic acid | 0.75 |
| Water | ad 100.0 |

The pH of the composition is 3.5. The color direction is red.

The test is designated as "Pair Home Use Test" and the results obtained are summarized in the table II below.

**Table II: Results of the Pair Home Use Test.**

| | **After 1 application** | **After 5 applications** | **After 10 applications** |
|---|---|---|---|
| **Wet hair** | | | |
| Combing | 4.0 | 4.1 | 4.2 |
| Softness | 4.3 | 4.4 | 4.5 |
| Nice feel | 4.1 | 4.5 | 4.5 |
| | | | |

| **Dry hair** | | | |
|---|---|---|---|
| Combing | 3.9 | 4.0 | 4.1 |
| Softness | 3.9 | 4.3 | 4.5 |
| Nice feel | 4.1 | 4.5 | 4.5 |
| Volume | 4.3 | 4.4 | 4.4 |
| Shine | 4.4 | 4.7 | 4.8 |
| Body | 4.2 | 4.5 | 4.5 |
| Managability | 4.3 | 4.6 | 4.7 |
| Want to buy | 4.3 | 4.4 | 4.8 |

From the results above, it is clear that in all items the pair use resulted in superior performance compared to the performance of the conditioner when used with a "normal" shampoo composition having a pH value 5 or above.

Accordingly the present invention is as well on the process of improvement of color, shine, manageability, softness, combing, volume, body, nice felling on touching of hair by a process wherein first a shampoo composition comprising at least one cleansing surfactant, at least one cationic direct dyestuff and at least one dicarboxylic and/or hydroxycarboxylic acid is applied to hair and rinsed off and subsequently a composition according to the present invention, namely a conditioning composition comprising at least one hair conditioning compound, at least one cationic direct dye and at least one dicarboxylic and/or hydroxycarboxylic acid is applied to hair, wherein both composition have a pH value below 4.5, preferably in the range of 2.0 to 4.0, more preferably 2.5 to 4.0, most preferably 2.5 to 3.80.

### Example 2

### Foam conditioner

| | |
|---|---|
| Quaternium-80 | 0.2 (Gew.-%) |
| Polyquaternium-11 | 0.7 |
| PEG-160-hydrogenated ricinus oil | 0,5 |
| Fragrance, preservative | q.s. |
| | |
| Basic red 76 | 0.05 |
| Basic brown 16 | 0.15 |
| Basic blue 99 | 0.04 |
| | |
| Malic acid | 0.5 |
| Lactic acid | 0.5 |
| Wasser | ad 100.0 |

pH of the composition is adjusted to 3.0. The composition is suitable for leave-in and rinse off. In leave-in application, amount used is obviously less than in the case of a rinse of application. The colour direction on the hair is brown. The composition is packed into an aerosol can with 90/10 ratio, by weight, liquid composition to propellant. As propellant propane, butane mixture is used.

### Example 3

| | |
|---|---|
| Cetylstearylalcohol | 4.0 (% by weight) |
| Cetrimoniumchloride | 2.0 |
| Panthenol | 0,4 |
| Dimethicone | 0.75 |
| Hydroxypropyl Guar Hydroxypropyltrimonium Chloride | 1.0 |
| Avocado extract | 0.5 |
| Fragrance, preservative | q.s. |
| Malic acid | 0.8 |
| Basic red 51 | 0.1 |
| Wasser | ad 100.0 |

The pH of the composition is 3.3. Colour direction on the hair is red.

### Example 4

| | |
|---|---|
| Cetylstearylalcohol | 5.0 (% by weight) |
| Steartrimonium Chloride | 0.5 |
| Behentrimonium Chloride | 0.4 |
| Mineral Oil( Paraffinum liquidum) | 0.5 |
| Isopropyl Myrisate | 0.3 |
| Bamboo extract | 0.3 |
| Fragrance, preservative | q.s. |
| Malic acid | 1.0 |
| Basic yellow 71 | 0.05 |
| Basic red 76 | 0.002 |
| Wasser | add 100.0 |

pH of the composition is 2.8. Colour direction on the hair is warm blond.

### Example 5

| | |
|---|---|
| Cetylstearylalcohol | 5.0 (% by weight) |
| Steartrimonium Chloride | 0.5 |
| Behentrimonium Chloride | 0.4 |
| Mineral Oil( Paraffinum liquidum) | 0.5 |
| Isopropyl Myrisate | 0.3 |
| Bamboo extract | 0.3 |
| Fragrance, preservative | q.s. |
| Malic acid | 0.5 |
| Lactic acid | 0.3 |
| Basic Blue 99 | 0.0005 |
| Basic red 51 | 0.00002 |
| Wasser | add 100.0 |

pH of the composition is 3.2. The composition has a superior performance on gray hair in terms of colour equalizing and as well as show anti-yellow effect on blond and bleached hair.

## Claims

1. Conditioning and colouring composition for hair **characterised in that** it comprises at least one hair conditioning compound, at least one cationic hair dye and at least one hydroxycarboxylic acid and/or dicarboxylic acid and have a pH lower than 4.5.

2. Composition according to claim 1 **characterised in that** conditioning compound is a cationic surfactant according to formula 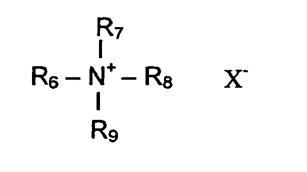 where R₆ is a saturated or unsaturated, branched or non-branched alkyl chain with 8-22 C atoms or
R₁₀ CO NH (CH₂)ₙ
where R₁₀ is saturated or unsaturated, branched or non-branched alkyl chain with 7-21 C atoms and n has typical value of 0 - 4 or
R₁₁ CO O (CH₂)ₙ
where R₁₁ is saturated or unsaturated, branched or non-branched alkyl chain with 7-21 C atoms and n has typical value of 0 - 4, and
R₇ is a hydrogen, lower alkyl chain with 1 to 4 carbon atoms, saturated or unsaturated, branched or non-branched alkyl chain with 8-22 C atoms or
R₁₀ CO NH (CH₂)ₙ
where R₁₀ is saturated or unsaturated, branched or non-branched alkyl chain with 7-21 C atoms and n has typical value of 0 - 4 or
R₁₁ CO O (CH₂)ₙ
where R₁₁ is saturated or unsaturated, branched or non-branched alkyl chain with 7-21 C atoms and n has typical value of 0 - 4, and
R₈ and R₉ are independent from each other H or lower alkyl chain with 1 to 4 carbon atoms, and X is chloride, bromide or methosulfate.

3. Composition according to claim 1 **characterised in that** conditioning compound is a cationic polymer.

4. Composition according to any of the preceding claims **characterized in that** it has a pH in the range of 2.0 to 4.0.

5. Composition according to any of the preceding claims **characterized in that** it has a pH in the range of 2.5 to 3.8.

6. Composition according to any of the preceding claims **characterized in that** concentration of cationic direct dye is in the range of 0.0001 to 1.5% by weight calculated to the total composition.

7. Composition according any of the preceding claims **characterized in that** concentration of at least one dicarboxylic acid and/or hydroxycarboxylic acid is in the range of 0.1 to 5% by weight calculated to the total composition.

8. Composition according any of the preceding claims **characterized in that** it comprises malic acid or lactic acid as a hydroxycarboxylic and/or dicarboxylic acid.

9. Composition according any of the preceeding claims **characterized in that** it comprises dicarboxylic acid and hydroxycarboxylic acid at concentration of 0.1 to 5% by weight with the condition that it comprises malic acid at a concentration of not less than 0.5% by weight calculated to total composition.

10. Composition according any of the preceding claims **characterized in that** it comprises only malic acid.

11. Composition according any of the preceding claims **characterized in that** it comprises organic solvents at a concentration of less than 10% by weight calculated to total concentration.

12. Conditioning composition according to any of the preceding claims **characterised in that** the conditioning composition is an emulsion and comprises at least one fatty alcohol.

13. Use of composition according to any of the preceding claims for improving color, shine, manageability, softness, combing, volume, body and nice felling on touching of hair.

14. Process for improvement of color, shine, manageability, softness, combing, volume, body, nice felling on touching of hair **characterised in that** first a shampoo composition comprising at least one cleansing surfactant, at least one cationic direct dyestuff and at least one dicarboxylic and/or hydroxycarboxylic acid is applied to hair and rinsed off and subsequently a conditioning composition comprising at least one hair conditioning compound, at least one cationic direct dye and at least one dicarboxylic and/or hydroxycarboxylic acid is applied to hair, wherein both composition have a pH value below 4.5, preferably in the range of 2.0 to 4.0, more preferably 2.5 to 4.0, most preferably 2.5 to 3.80 and rinsed off.

## Patentansprüche

1. Konditionierende und färbende Zusammensetzung für Haare, **dadurch gekennzeichnet, dass** sie mindestens eine konditionierende Verbindung, mindestens eine kationische Haarfarbe und mindestens eine Hydroxycarboxylsäure und/oder Dicarboxylsäure enthält und einen pH- Wert von weniger als 4,5 hat.

2. Zusammensetzung nach Anspruch 1, **dadurch gekennzeichnet, dass** die konditionierende Verbindung ein kationisches Tensid entsprechend der Formel 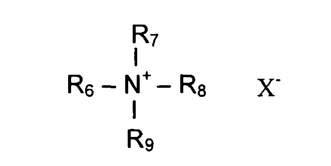 ist,
worin R₆ eine gesättigte oder ungesättigte, verzweigte oder nicht verzweigte Alkylkette mit 8-22 C- Atomen ist, oder
R₁₀ CO NH (CH₂)ₙ
worin R₁₀ eine gesättigte oder ungesättigte, verzweigte oder nicht verzweigte Alkylkette mit 7-21 C- Atomen ist und n einen typischen Wert von 0-4 hat, oder
R₁₁ CO O (CH₂)ₙ
worin R₁₁ eine gesättigte oder ungesättigte, verzweigte oder nicht verzweigte Alkylkette mit 7 - 21 C- Atomen ist und n einen typischen Wert von 0 - 4 hat und
R₇ Wasserstoff, eine niedrige Alkylkette mit 1 -4 Kohlenstoffatomen, eine gesättigte oder ungesättigte, verzweigte oder nicht verzweigte Alkylkette mit 8 - 22 C- Atomen ist, oder
R₁₀ CO NH (CH₂)ₙ
worin R₁₀ eine gesättigte oder ungesättigte, verzweigte oder nicht verzweigte Alkylkette mit 7 - 21 C- Atomen ist und n einen typischen Wert vo 0 - 4 hat, oder
R₁₁ CO O (CH₂)ₙ
worin R₁₁ eine gesättigte oder ungesättigte, verzweigte oder nicht verzweigte Alkylkette mit 7-21 C- Atomen ist und n einen typischen Wert von 0-4 hat, und
R₈ und R₉ unabhängig voneinander H oder niedrige Alkylketten mit 1 - 4 C- Atomen sind, und X Chlorid, Bromid oder Methosulfat ist.

3. Zusammensetzung nach Anspruch 1, **dadurch gekennzeichnet, dass** die konditionierende Verbindung ein kationisches Polymer ist.

4. Zusammensetzung nach einem der vorhergehenden Ansprüche, **dadurch gekennzeichnet, dass** sie einen pH- Wert im Bereich von 2,0 bis 4,0 hat.

5. Zusammensetzung nach einem der vorhergehenden Ansprüche, **dadurch gekennzeichnet, dass** sie einen pH- Wert im Bereich von 2,5 bis 3,8 hat.

6. Zusammensetzung nach einem der vorhergehenden Ansprüche, **dadurch gekennzeichnet, dass** die Menge der direkt ziehenden kationischen Farbe im Bereich von 0,0001 bis 1,5 Gew.- %, berechnet auf die Gesamtzusammensetzung, liegt.

7. Zusammensetzung nach einem der vorhergehenden Ansprüche, **dadurch gekennzeichnet, dass** die Menge von mindestens einer Dicarboxylsäure und/oder Hydroxycarboxylsäure im Bereich von 0,1 bis 5 Gew.- %, berechnet auf die Gesamtzusammensetzung, liegt.

8. Zusammensetzung nach einem der vorhergehenden Ansprüche, **dadurch gekennzeichnet, dass** sie Apfelsäure oder Milchsäure als Hydroxycarboxylsäure und/oder Dicarboxylsäure enthält.

9. Zusammensetzung nach einem der vorhergehenden Ansprüche, **dadurch gekennzeichnet, dass** sie Dicarboxylsäure und Hydroxycarboxylsäure in einer Menge von 0,1 bis 5 Gew.- % enthält, mit der Bedingung, dass sie Apfelsäure in einer Menge von nicht weniger als 0,5 Gew.- %, berechnet auf die Gesamtzusammensetzung, enthält.

10. Zusammensetzung nach einem der vorhergehenden Ansprüche, **dadurch gekennzeichnet, dass** sie nur Apfelsäure enthält.

11. Zusammensetzung nach einem der vorhergehenden Ansprüche, **dadurch gekennzeichnet, dass** sie organische Lösungsmittel in einer Menge von weniger als 10 Gew.- %, berechnet auf die Gesamtzusammensetzung, enthält.

12. Konditionierende Zusammensetzung nach einem der vorhergehenden Ansprüche, **dadurch gekennzeichnet, dass** die konditionierende Zusammensetzung eine Emulsion ist und mindestens einen Fettalkohol enthält.

13. Verwendung der Zusammensetzung nach einem der vorhergehenden Ansprüche zum Verbessern der Farbe, Leuchtkraft, Bearbeitung, Weichheit, Kämmbarkeit, Volumen, Körper und gutem Feeling beim Berühren der Haare.

14. Verfahren zur Verbesserung der Farbe, Leuchtkraft, Bearbeitung, Weichheit, Kämmbarkeit, Volumen, Körper, gutem Feeling beim Berühren der Haare, **dadurch gekennzeichnet, dass** zuerst ein Shampoo das mindestens ein reinigendes Tensid, mindestens einen kationischen direkt ziehenden Farbstoff und mindestens eine Dicarboxy- und/oder Hydroxycarboxylsäure enthält auf das Haar aufgebracht und wieder abgespült wird und anschließend eine konditionierende Zusammensetzung die mindestens eine konditionierende Verbindung, mindestens eine kationische direkt ziehende Farbe und mindestens eine Dicarboxy- und/oder Hydroxycarboxylsäure enthält auf das Haar aufgebracht und ausgespült wird, wobei beide Zusammensetzungen einen pH- Wert unter 4,5, vorzugsweise im Bereich von 2,0 bis 4,0, besonders bevorzugt 2,5 bis 4,0, ganz besonders bevorzugt 2,5 bis 3,8 haben.

## Revendications

1. Composition de conditionnement et de coloration pour les cheveux **caractérisée en ce qu'**elle comprend au moins un composé de conditionnement des cheveux, au moins un colorant capillaire cationique et au moins un acide hydroxycarboxylique et/ou un acide dicarboxylique et a un pH inférieur à 4,5.

2. Composition selon la revendication 1 **caractérisée en ce que** le composé de conditionnement est un agent tensioactif cationique selon la formule 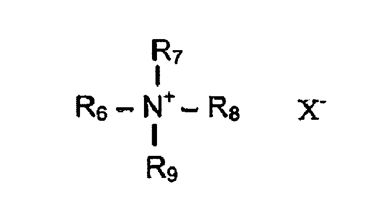 où R₆ est une chaîne alkyle saturée ou insaturée, ramifiée ou non ramifiée avec 8 à 22 atomes de C ou
R₁₀CONH(CH₂)ₙ
où R₁₀ est une chaîne alkyle saturée ou insaturée, ramifiée ou non ramifiée avec 7 à 21 atomes de C et n a une valeur type de 0 à 4 ou
R₁₁COO(CH₂)ₙ
où R₁₁ est une chaîne alkyle saturée ou insaturée, ramifiée ou non ramifiée avec 7 à 21 atomes de C et n a une valeur type de 0 à 4, et
R₇ est un hydrogène, une chaîne alkyle inférieure avec 1 à 4 atomes de carbone, une chaîne alkyle saturée ou insaturée, ramifiée ou non ramifiée avec 8 à 22 atomes de C ou
R₁₀CONH(CH₂)ₙ
où R₁₀ est une chaîne alkyle saturée ou insaturée, ramifiée ou non ramifiée avec 7 à 21 atomes de C et n a une valeur type de 0 à 4 ou
R₁₁COO(CH₂)ₙ
où R₁₁ est une chaîne alkyle saturée ou insaturée, ramifiée ou non ramifiée avec 7 à 21 atomes de C et n a une valeur type de 0 à 4, et
R₈ et R₉ sont indépendamment l'un de l'autre H ou une chaîne alkyle inférieure avec 1 à 4 atomes de carbone, et X est un chlorure, un bromure ou un méthosulfate.

3. Composition selon la revendication 1 **caractérisée en ce que** le composé de conditionnement est un polymère cationique.

4. Composition selon l'une quelconque des revendications précédentes **caractérisée en ce qu'**elle a un pH dans la plage de 2,0 à 4,0.

5. Composition selon l'une quelconque des revendications précédentes **caractérisée en ce qu'**elle a un pH dans la plage de 2,5 à 3,8.

6. Composition selon l'une quelconque des revendications précédentes **caractérisée en ce que** la concentration du colorant direct cationique est dans la plage de 0,0001 à 1,5% en poids calculée sur la composition totale.

7. Composition selon l'une quelconque des revendications précédentes **caractérisée en ce que** la concentration d'au moins un acide dicarboxylique et/ou un acide hydroxycarboxylique est dans la plage de 0,1 à 5% en poids calculée sur la composition totale.

8. Composition selon l'une quelconque des revendications précédentes **caractérisée en ce qu'**elle comprend de l'acide malique ou de l'acide lactique en tant qu'acide hydroxycarboxylique et/ou dicarboxylique.

9. Composition selon l'une quelconque des revendications précédentes **caractérisée en ce qu'**elle comprend de l'acide dicarboxylique et de l'acide hydroxycarboxylique à une concentration de 0,1 à 5% en poids à condition qu'elle comprenne de l'acide malique à une concentration de pas moins de 0,5% en poids calculée sur la composition totale.

10. Composition selon l'une quelconque des revendications précédentes **caractérisée en ce qu'**elle comprend uniquement de l'acide malique.

11. Composition selon l'une quelconque des revendications précédentes **caractérisée en ce qu'**elle comprend des solvants organiques à une concentration de moins de 10% en poids calculée sur la composition totale.

12. Composition de conditionnement selon l'une quelconque des revendications précédentes **caractérisée en ce que** la composition de conditionnement est une émulsion et comprend au moins un alcool gras.

13. Utilisation d'une composition selon l'une quelconque des revendications précédentes pour améliorer la couleur, le brillant, la maniabilité, la douceur, le coiffage, le volume, le corps et la bonne impression au toucher des cheveux.

14. Procédé pour l'amélioration de la couleur, du brillant, de la maniabilité, de la douceur, du coiffage, du volume, du corps et de la bonne impression au toucher des cheveux **caractérisé en ce que** premièrement une composition de shampoing comprenant au moins un agent tensioactif nettoyant, au moins une matière colorante directe cationique et au moins un acide dicarboxylique et/ou hydroxycarboxylique est appliquée aux cheveux et rincée et ensuite une composition de conditionnement comprenant au moins un composé de conditionnement des cheveux, au moins un colorant direct cationique et au moins un acide dicarboxylique et/ou hydroxycarboxylique est appliquée aux cheveux, dans lequel les deux compositions ont une valeur de pH inférieure à 4,5, préférablement dans la plage de 2,0 à 4,0, plus préférablement 2,5 à 4,0, le plus préférablement 2,5 à 3,80, et rincée.
